(19) Europäisches Patentamt
European Patent Office
Office européen des brevets

(11) Veröffentlichungsnummer: **0 277 554 B1**

# (12) EUROPÄISCHE PATENTSCHRIFT

(45) Veröffentlichungstag der Patentschrift: **17.04.91 Patentblatt 91/16**

(51) Int. Cl.5: **C07C 249/12,** C07C 251/32

(21) Anmeldenummer: **88100855.1**

(22) Anmeldetag: **21.01.88**

## (54) Verfahren zur Herstellung von Hydroxybenzaldoxim-O-ethern.

(30) Priorität: **04.02.87 DE 3703236**

(43) Veröffentlichungstag der Anmeldung: **10.08.88 Patentblatt 88/32**

(45) Bekanntmachung des Hinweises auf die Patenterteilung: **17.04.91 Patentblatt 91/16**

(84) Benannte Vertragsstaaten: **BE CH DE FR GB IT LI NL**

(56) Entgegenhaltungen:
**EP-A- 0 229 641**
**HOUBEN-WEYL; "Methoden der organischen chemie", Band VI/1c, Teil 1, 1976, Seiten 247-249, Georg Thieme Verlag, Stuttgart, DE**

(56) Entgegenhaltungen:
**CHEMICAL AND PHARMACEUTICAL BULLETIN, Band 29, Nr. 5, Mai 1981, Seiten 1443-1445, Tokyo, JP; T. SATOH et al.: "Selective reduction of aromatic nitro compounds with sodium borohydride-stannous chloride"**

(73) Patentinhaber: **BAYER AG**
**W-5090 Leverkusen 1 Bayerwerk (DE)**

(72) Erfinder: **Lantzsch,Reinhard, Dr.**
**Am Buschhäuschen 51**
**W-5600 Wuppertal 1 (DE)**
Erfinder: **Ziemann, Heinz, Dr.**
**Am Wiesenberg 10**
**W-5653 Leichlingen (DE)**
Erfinder: **Elbe, Hans-Ludwig, Dr.**
**Dasnöckel 59**
**W-5600 Wuppertal 11 (DE)**

EP 0 277 554 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein neues Verfahren zur Herstellung von bekannten Hydroxybenzaldoxim-O-ethern, die als Zwischenprodukte zur Synthese von Verbindungen mit fungizider, insektizider und antimykotischer Wirksamkeit verwendet werden können.

Es ist bereits bekannet geworden, daß man bestimmte Hydroxy-benzaldoxim-O-ether herstellen kann, indem man Hydroxybenzaldehyde mit den entsprechenden Hydroxylamin-Derivaten umsetzt (vgl. EP-OS 0 076 370 und EP-OS 0 115 828). Die betreffende Umsetzung läßt sich durch das folgende Reaktionsschema veranschaulichen :

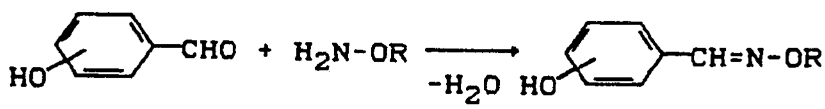


R = Alkyl, Alkenyl, Alkinyl.

Nachteilig an diesem Verfahren ist jedoch, daß die Ausgangsstoffe nur durch aufwendige Synthese zugänglich sind. Sie sind deshalb relativ kostspielig und ihr Einsatz für eine Herstellung von Hydroxy-benzaldoxim-O-ethern in technischem Maßstab ist problematisch. Außerdem sind die Ausbeuten an Hydroxy-benzaldoxim-O-ethern bei dem oben angegebenen Verfahren nicht immer befriedigend.

Es wurde nun gefunden, daß man bekannte Hydroxy-benzaldoxim-O-ether der Formel

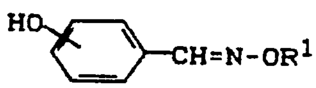 (I)

in welcher
$R^1$ für Alkyl steht,
erhält, wenn man

a) in einer ersten Stufe Nitro-benzaldoxim-O-ether der Formel

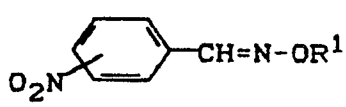 (II)

in welcher
$R^1$ die oben angegebene Bedeutung hat,
mit der berechneten Menge Wasserstoff in Gegenwart eines Katalysators sowie in Gegenwart eines Verdünnungsmittels hydriert und

b) in einer zweiten Stufe die so erhaltenen Aminobenzaldoxim-O-ether der Formel

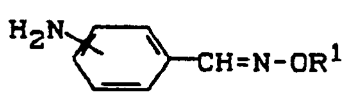 (III)

in welcher
$R^1$ die oben angegebene Bedeutung hat,

mit einem Diazotierungsmittel in saurer, wäßriger Lösung umsetzt und die entstehenden Diazoniumsalze der Formel

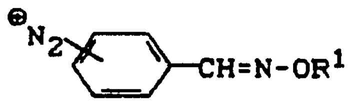 $X^{\ominus}$ (IV)

in welcher

$X^{\ominus}$ für ein Äquivalent eines anorganischen Anions steht und

$R^1$ die oben angegebene Bedeutung hat,

ohne Zwischenisolierung in saurer, wäßriger Lösung thermisch hydrolysiert.

Es ist als äußerst überraschend zu bezeichnen, daß die erfindungsgemäße Umsetzung unter den angegebenen Verfahrensbedingungen glatt und in guter Ausbeute abläuft. Aufgrund des bekannten Standes der Technik war nämlich zu erwarten, daß bei der Hydrierung in der ersten Stufe des erfindungsgemäßen Verfahrens die in den Verbindungen der Formel (II) verhandene C = N-Doppelbindung zu einem beträchtlichen Teil abgesättigt wird, denn Hydrierungen solcher Doppelbindungen erfolgen sehr leicht (vgl. Chem. ber. 88, 38 (1955) ; J. Amer. Chem. Soc. 59, 716 (1937) und J. Amer. Chem. Soc. 52, 669 (1930). Unter Umständen erfolgt sogar eine Reduktion bis zum Amin oder eine Hydrogenolyse bis zur Methylgruppe.

Ferner ist bekannt, daß Oxime durch Reduktionsmittel in Gegenwart von Raney-Nickel in Aldehyde überführt werden können (vgl. Chem. Comm. 16, 803 (1986). Es war daher anzunehmen, daß eine derartige hydrogenolytische Spaltung auch unter den Umsetzungsbedingungen in der ersten Stufe des erfindungsgemäßen Verfahrens eintritt. Außerdem war zu befürchten, daß eine Hydrolyse des Oximethers durch das bei der Reduktion der Nitrogruppe entstehende Wasser zur Bildung eines Aldehyds führt. Überraschenderweise werden diese störenden Nebenreaktion im Falle des erfindungsgemäßen Verfahrens jedoch nicht beobachtet. Außerdem konnte auch nicht angenommen werden, daß in der zweitem Stufe des erfingungsgemäßen Verfahrens des bei der thermischen Hydrolyse des Diazoniumsalzes auftretende reaktive Carbo-Kation (am Aromaten) selektiv in der gewünschten Art und Weise reagiert und kein intermolekularer Angriff am freien Elektronenpaar des Oximether-Stickstoffs erfolgt.

Das erfindungsgemäße Verfahren zeichnet sich durch eine Reihe von Vorteilen aus. So ermöglicht es die Herstellung von Hydroxy-benzaldoxim-O-ethern der Formel (I) in hohen Ausbeuten, wobei billige und gut zugängliche Verbindungen als Ausgangsprodukte eingesetzt werden. Ferner ist die Umsetzung einfach durchführbar und die Isolierung der Benzaldoxim-O-ether der Formel (I) bereitet keinerlei Schwierigkeiten. Das erfindungsgemäße Verfahren ist daher besonders geeignet, um Hydroxy-benzaldoxim-O-ether der Formel (I) in technischem Maßstab herzustellen.

Verwendet man 4-Nitro-benzaldoxim-O-methylether als Ausgangsstoff, Wasserstoff als Hydrierungsmittel, Raney-Nickel als Katalysator, wäßrige Schwefelsäure als saures Medium und Natriumnitrit als Diazotierungsmittel, so kann der Verlauf des erfindungsgemäßen Verfahrens durch das folgende Formelschema veranschaulicht werden :

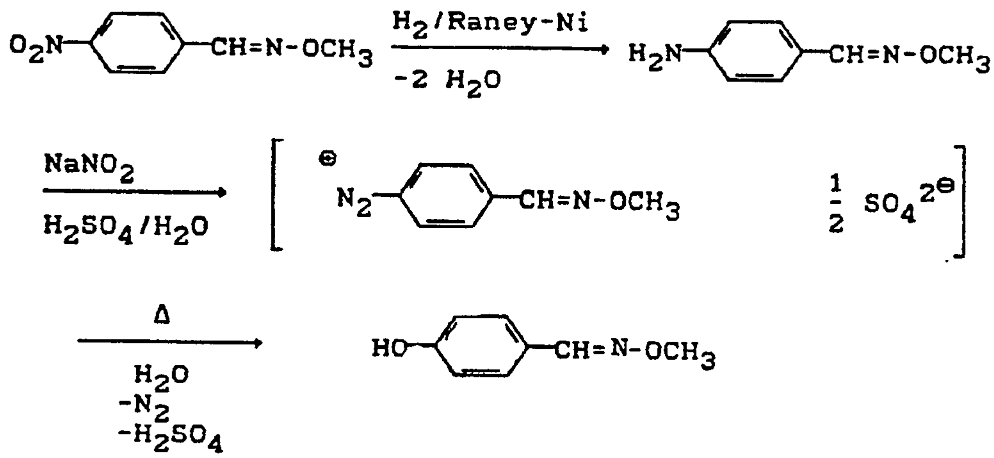


Die bei dem erfindungsgemäßen Verfahren als Ausgangsstoffe benötigten Nitro-benzaldoxim-O-ether sind durch die Formel (II) allgemein definiert. In dieser Formel steht $R^1$ vorzugsweise für geradkettiges oder verzweigtes Alkyl mit 1 bis 20 Kohlenstoffatomen.

Besonders bevorzugte Ausgangsstoffe sind diejenigen Nitro-benzaldoxim-O-ether der Formel (II) in denen $R^1$ für geradkettiges oder verzweigtes Alkyl mit 1 bis 10 Kohlenstoffatomen steht.

Ganz besonders bevorzugte Ausgangsstoffe sind diejenigen Nitro-benzaldoxim-O-ether der Formel (II), in denen $R^1$ für geradkettiges oder verzweigtes Alkyl mit 1 bis 4 Kohlenstoffatomen steht, wobei die 4-Nitro-benzaldoxim-O-ether jeweils von speziellem Interesse sind.

Die Nitro-benzaldoxim-O-ether der Formel (II) sind bekannt oder lassen sich nach bekannten Verfahren in einfacher Weise herstellen (vgl. Ber. 24, 2548 (1891)).

In der ersten Stufe des erfingungsgemäßen Verfahrens dient Wasserstoff als Hydrierungsmittel. Dabei kann der Wasserstoffdruck innerhalb eines größeren Bereiches variiert werden. Im allgemeinen arbeitet man unter einem Wasserstoffdruck zwischen 1 und 50 bar, vorzugsweise zwischen 2 und 20 bar, insbesondere zwischen 3 und 10 bar.

Als Katalysatoren kommen bei der Durchführung der ersten Stufe des erfingungsgemäßen Verfahrens vorzugsweise nickelhaltige Katalysatoren in Frage wie z.B. Raney-Nickel, Raney-Nickel-Eisen, Raney-Nickel-Kupfer oder Nickel auf Siliciumdioxid.

Als Verdünnungsmittel kommen bei der Durchführung der ersten Stufe des erfingungsgemäßen Verfahrens alle für derartige Umsetzungen üblichen inerten organischen Solventien in Betracht. Vorzugsweise verwendbar sind Ether, wie Diethylether, Dioxan oder Tetrahydrofuran, ferner Alkohole, wie Methanol, Ethanol oder Isopropanol, und außerdem aliphatische, cycloaliphatische oder aromatische Kohlenwasserstoffe, wie Pentan, Hexan, Petrolether, Cyclohexan, Methylcyclohexan, Benzol, Toluol oder Xylol, ferner Ester und Amide, wie z.B. Dimethylformamid.

Die Reaktionstemperaturen können bei der Durchführung die ersten Stufe des erfindungsgemäßen Verfahrens innerhalb eines bestimmten Bereiches variiert werden. Im allgemeinen arbeitet man bei Temperaturen zwischen 0°C und 150°C, vorzugsweise zwischen 5°C und 50°C, insbesondere zwischen 10 und 30°C.

Als Diazotierungsmittel kommen bei der Durchführung der zweiten Stufe des erfindungsgemäßen Verfahrens alle üblicherweise für eine Diazotierung geeigneten Komponenten in Frage. Vorzugsweise verwendbar sind Natriumnitrit, Kaliumnitrit, Ammoniumnitrit sowie Nitrosylschwefelsäure.

Die Umsetzung in der zweiten Stufe des erfindungsgemäßen Verfahrens wird in saurer, wäßriger Lösung durchgeführt. Als Säuren kommen vorzugsweise anorganische Säuren in Frage. Besonders bevorzugt verwendbar ist Schwefelsäure. Demgemäß steht $X^{\ominus}$ im Falle der Diazonium-Salze der Formel (IV) für ein Äquivalent eines Anions der verwendeten anorganischen Säure Vorzugsweise steht $X^{\ominus}$ für ein Äquivalent eines Sulfatanions.

Bei der Diazotierung kann die Konzentration an Säure innerhalb eines größeren Bereiches variiert werden. Im allgemeinen arbeitet man mit Lösungen, in denen die Säurekonzentration zwischen 1 und 50 Gew.%, vorzugsweise zwischen 1 und 30 Gew.% liegt.

Auch bei der Durchführung der zweiten Stufe des erfindungsgemäßen Verfahrens können die Reaktionstemperaturen innerhalb eines bestimmten Bereiches variiert werden. Bei der Diazotierung arbeitet man im allgemeinen bei Temperaturen zwischen 0 und 20°C, vorzugsweise zwischen 0 und 10°C.

Die anschließende thermische Hydrolyse der Diazoniumsalze wird im allgemeinen bei Temperaturen zwischen 50 und 100°C, vorzugsweise zwischen 70 und 100°C durchgeführt.

Bei der Durchführung des erfindungsgemäßen Verfahrens setzt man in der ersten Stufe Nitrobenzaldoxim-O-ether der Formel (II) mit der berechneten Menge an Wasserstoff in Gegenwart eines Katalysators um.

Bei der Durchführung der zweiten Stufe des erfindungsgemäßen Verfahrens setzt man auf 1 Mol Aminobenzaldoxim O-ether der Formel (II) im allgemeinen 1 bis 1,5 Mol, vorzugsweise 1 bis 1,2 Mol Diazotierungsmittel ein. Das überschüssige Diazotierungsmittel kann in üblicher Weise vor der thermischen Hydrolyse durch Zugabe von Harnstoff zerstört werden.

In einer besonderen Ausführungsform der zweiten Stufe des erfindungsgemäßen Verfahrens wird die Diazoniumsalzlösung zur thermischen Hydrolyse in eine 50 bis 100°C, vorzugsweise 70 bis 100°C heiße wäßrige Schwefelsäure eindosiert, wobei die Konzentration der Schwefelsäure 1 bis 50 Gew.-%, vorzugsweise 1 bis 30 Gew.-% beträgt. Die Eindosier-Geschwindigkeit wird dabei so gewählt, daß die Temperatur der vorgelegten wäßrigen Schwefelsäure konstant gehalten werden kann.

Im einzelnen geht man bei der Durchführung der ersten Stufe des erfindungsgemäßen Verfahrens so vor, daß man Nitro-benzaldoxim-O-ether, Katalysator und Verdünnungsmittel in ein Druckgefäß gibt und dann so lange Wasserstoff zupumpt, bis die theoretisch erforderliche Menge aufgenommen ist. Danach arbeitet man auf, indem man den Katalysator abfiltriert, das Filtrat unter vermindertem Druck einengt und gegebenenfalls das verbleibende Produkt durch Destillation reinigt. Bei der Durchführung der zweiten Stufe des erfindungsgemäßen Verfahrens geht man im allgemeinen so vor, daß man Amino-benzaldoxim-O-ether in verdünnter, wäßriger Säure aufschlämmt, das Reaktionsgemisch kühlt, mit dem Diazotierungsmittel versetzt und nach beendeter Umsetzung gegebenenfalls nach vorhandenes Diazotierungsmittel durch Zugabe von Harnstoff zerstört. Zur anschließenden thermischen Hydrolyse gibt man die Diazoniumsalz-Lösung in erwärmte säure und läßt noch einige Minuten nachrühren. Die Isolierung der Endprodukte erfolgt nach üblichen Methoden. Im allgemeinen geht man so vor, daß man das Reaktionsgemisch auf Raumtemperatur abkühlt, durch Zugabe von Base auf einen pH-Wert im alkalischen Bereich einstellt, mehrfach mit einem in Wasser wenig löslichen organischen Solvens extrahiert, die vereingten organischen Phasen gegebenenfalls nach vorherigem trocknen unter vermindertem Druck einengt und das verbleibende Produkt unter vermindertem Druck destilliert.

Die nach dem erfindungsgemäßen Verfahren herstellbaren Hydroxy-benzaldoxim-O-ether der Formel (I) sind allgemein wertvolle Ausgangsstoffe zur Synthese von biologisch aktiven Verbindungen. So lassen sie sich z.B. verwenden zur Synthese von Oximethern, welche gute insektizide Eigenschaften besitzen (vgl. EP-OS 0 115 828) ; von Azolylphenoxy-Derivaten, welche hervorragende fungizide Eigenschaften aufweisen (vgl. EP-OS 076 370) ; von 1-Hydroxyethyl-triazolyl-Derivaten, welche gute fungizide und antimykotische Eigenschaften aufweisen (vgl. EP-OS 0 110 048 und DE-OS 3 314 548) ; sowie von Hydroxyalkylazolyl-Derivaten, welche gute antimykotische Wirksamkeit besitzen (vgl. DE-OS 3 427 844).

So Läßt sich beispielsweise das 3,3-Dimethyl-1-(4-methoximinomethyl-phenoxy)-1-(1,2,4-triazol-1-yl)-butan-2-on der Formel

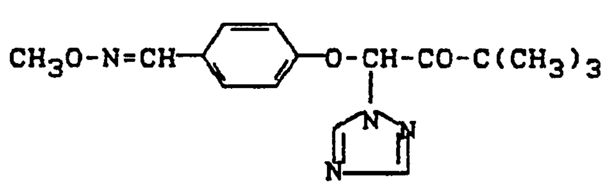

herstellen, indem man 3,3-Dimethyl-1-(1,2,4-triaxol-1-yl)-butan-2-on zunächst mit Brom zum 1-Brom-(1,2,4-triazol-1-yl)-3,3-dimethyl-butan-2-on umsetzt und dieses anschliessend mit 4-Hydroxybenzaldehyd-O-methyloximether in Gegenwart einer Base umsetzt. Diese Synthese läßt sich formelwäßig wie folgt veranschaulichen:

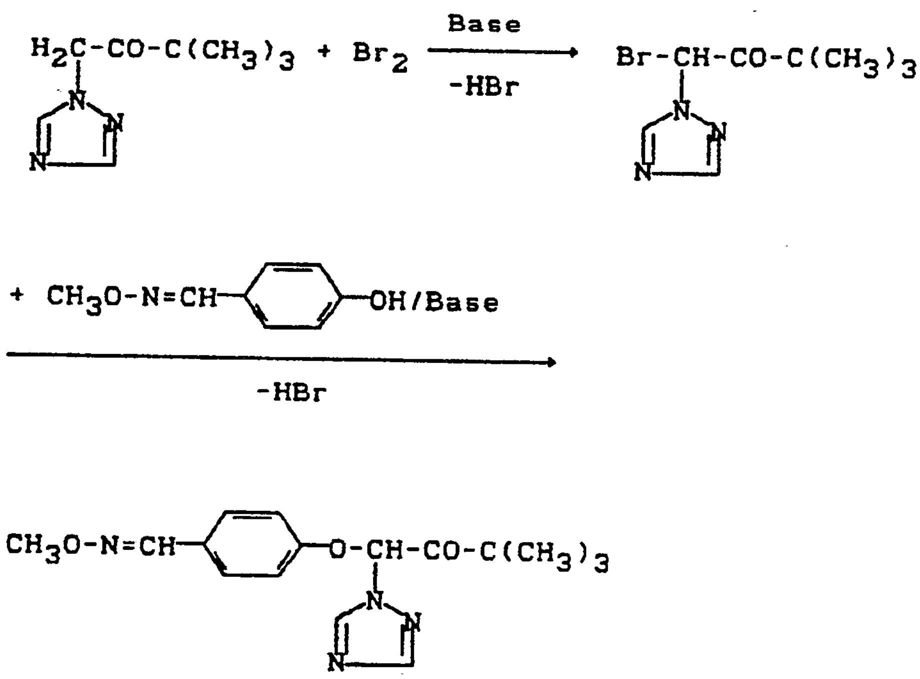


Das erfindungsgemäße Verfahren wird durch das nachfolgende Beispiel veranschaulicht.

Beispiel 1

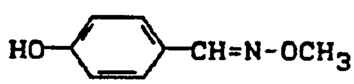 (I-1)

1. Stufe

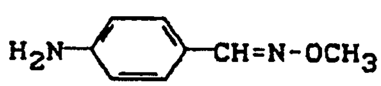 (III-1)

Eine Lösung von 45 g (0,25 Mol) 4-Nitro-benzaldoxim-O-methylether in 450 ml Tetrahydrofuran wird in ein Druckgefäß gegeben und mit 7,5 g Raney-Nickel versetzt. Es wird so lange Wasserstoff bei Raumtemperatur zugeführt, bis die theoretisch berechnete Menge aufgenommen ist. Der Wasserstoff-Druck beträgt 3 bar. Man arbeitet danach auf, indem man des Reaktionsgemisch filtriert und das verbleibende Filtrat durch Abziehen des Lösungsmittels unter vermindertem Druck einengt. Der Rückstand wird einer Vakuumdestillation unterworfen. Man erhält auf diese Weise 38,1 g (95,2% der Theorie) an 4-Amino-benzaldoxim-O-methylether, Siedepunkt : 105°C/0,1 mbar.

2. Stufe

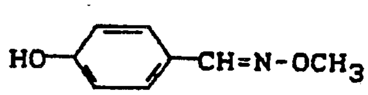 (I-1)

30 g (0,91 Mol) 4-Amino-benzaldoxim-O-methylether werden in 120 g 25%-iger wäßriger Schwefelsäure

aufgeschlämmt. Man läßt 30 Minuten bei Raumtemperatur nachrühren und kühlt sodann auf 5-10°C ab. Innerhalb von 30 Minuten läßt man eine Lösung von 13,8g (0,2 Mol) Natriumnitrit in 80 ml Wasser in das Reaktionsgemisch eintropfen.

Man rührt 1 Stund bei 5-10°C nach und zerstört anschließend überschüssiges Nitrit durch zugesetzten Harnstoff.

Zum Hydrolisieren läßt man die Diazoniumlösung rasch in vorgelegte 80-85°C heiße, wäßrige Schwegelsäure (120 g, 25%-ig) einlaufen, wobei die Temperatur konstant gehalten wird. Nach Beendigung der Zugabe läßt man 15 Minuten bei 80-85°C nachrühren. Zur Aufarbeitung wird auf Raumtemperatur abgekühlt, mit wäßriger Natronlauge auf pH 5-6 gestellt und mit Methylenchlorid extrahiert. Die organische Phase wird under vermindertem Druck eingeengt. Das verbleibende Rohprodukt wird im Vakuum destilliert. Man erhölt 25,7 g (Gehalt nach GC : 95,4% ; Ausbeute : 85% d Theorie) 4-Methoximinomethylphenol vom Siedepunkt 140 -142°C/0,13 mbar.

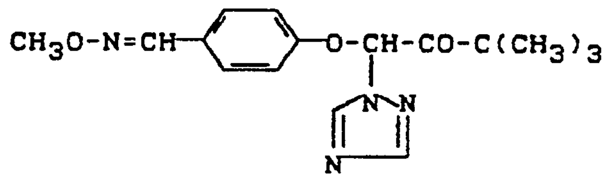

In eine Lösung von 217 g (1,3 Mol) 3,3-Dimethyl-1-(1,2,4-triazol-1-yl)-2-butanon in 700 ml Eisessig trägt man 110 g (1,34 Mol) Natriumacetat ein, wobei die Temperatur auf ca. 28°C ansteigt. Man läßt 30 Minuten nachrühren und versetzt tropfenweise unter leichter Kühlung bei 30 bis 33°C mit 208 g (1,3 Mol) Brom. Das Reaktionsgemisch wird 2,5 Stunden bei Raumtemperatur nachgerührt und in 1200 ml Wasser gegeben. Man extrahiert mit Methylenchlorid, wäscht mit Wasser und wäßriger Bicarbonatlösung, trocknet über Natriumsulfat und engt ein.

Das so erhaltene rohe 1-Brom-3,3-Dimethyl-1-(1,2,4-triazol-1-yl)-2-butanon wird in 100 ml Acetonitril gelöst und zu einer Aufschlämmung von 151 g (1 Mol) 4-Methoxyminomethylphenol und 150 g (1,09 Mol) Kaliumcarbonat in 800 ml Acetonitril gegeben, wobei die Temperatur auf etwa 40°C ansteigt. Man läßt das Reaktionsgemisch 3 Stunden bei 60 bis 65°C nachrühren, kühlt anschließend ab und gibt auf Wasser. Man extrahiert mit Toluol, wäscht mit Wasser, trocknet und engt. ein. Der Rückstand wird in Ligroin verrieben und auf Ton getrocknet. Man erhält 241 g (76% der Theorie) 3,3-Dimethyl-1-(4-methoximinomethyl-phenoxy)-1-(1,2,4-triazol-1-yl)-butan-2-on vom Schmelzpunkt 83-87°C.

Nach dem in Beispiel 1 beschriebenen Verfahren wird auch die in dem folgenden Beispiel aufgeführte Verbindung hergestellt.

Beispiel 2

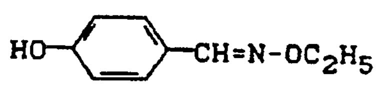

(I-2)

Schmelzpunkt : 64°C

## Ansprüche

1. Verfahren zur Herstellung von Hydroxy-benzaldoxim-O-ethern der Formel

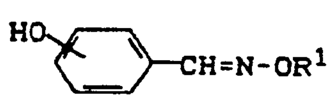

(I)

in welcher
$R^1$ für Alkyl steht,
dadurch gekennzeichnet, daß man
a) in einer ersten Stufe Nitro-benzaldoxim-O-ether der Formel

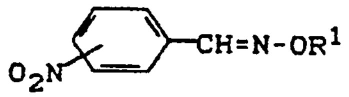

(II)

in welcher
$R^1$ die oben angegebene Bedeutung hat,
mit der berechneten Menge Wasserstoff in Gegenwart eines Katalysators sowie in Gegenwart eines Verdünnungsmittels hydriert und
b) in einer zweiten Stufe die so erhaltenen Amino-benzaldoxim-O-ether der Formel

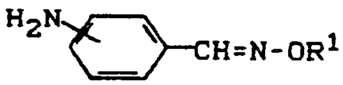

(III)

in welcher
$R^1$ die oben angegebene Bedeutung hat,
mit einem Diazotierungsmittel in saurer, wäßriger Lösung umsetzt und die entstehenden Diaxoniumsalze der Formel

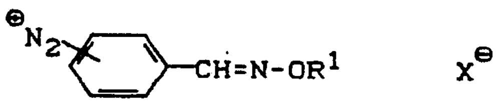

(IV)

in welcher
$X^{\ominus}$ für ein Äquivalent eines anorganischen Anions steht und
$R^1$die oben angegebene Bedeutung hat,
ohne Zwischenisolierung in saurer, wäßriger Lösung thermisch hydrolysiert.

2. Verfahren gemäß Anspruch 1, dadurch gekennzeichnet, daß man als Ausgangsstoffe Nitro-benzaldoxim-O-ether der Formel (II) einsetzt, in denen $R^1$ für geradkettiges oder verzweigtes Alkyl mit 1 bis 20 Kohlenstoffatomen steht.

3. Verfahren gemäß Anspruch 1, dadurch gekennzeichnet, daß man als Ausgangsstoffe Nitro-benzaldoxim-O-ether der Formel (II) einsetzt, in denen $R^1$ für geradkettiges oder verzweigtes Alkyl mit 1 bis 10 Kohlenstoffatomen steht.

4. Verfahren gemäß Anspruch 1, dadurch gekennzeichnet, daß man als Ausgangsstoffe Nitro-benzaldoxim-O-ether der Formel (II) einsetzt, in denen $R^1$ für geradkettiges oder verzweigtes Alkyl mit 1 bis 4 Kohlenstoffatomen steht.

5. Verfahren gemäß Anspruch 1, dadurch gekennzeichnet, daß man als Ausgangsstoff 4-Nitrobenzaldoxim-O-methylether einsetzt.

6. Verfahren gemäß Anspruch 1, dadurch gekennzeichnet, daß man nickelhaltige Katalysatoren als Reaktionsbeschleuniger einsetzt.

7. Verfahren gemäß Anspruch 1, dadurch gekennzeichnet, daß man bei der Durchführung der zweiten Stufe Natriumnitrit, Kaliumnitrit, Ammoniumnitrit oder Nitrosylschwefelsäure als Diazotierungsmittel einsetzt.

8. Verfahren gemäß Anspruch 1, dadurch gekennzeichnet, daß man bei der Durchführung der zweiten Stufe wäßrige Schwefelsäure als Acidifizierungsmittel einsetzt.

9. Verfahren gemäß Anspruch 1, dadurch gekennzeichnet, daß man bei der Durchführung der ersten Stufe

bei Temperaturen zwischen 0°C und 100°C arbeitet.

10. Verfahren gemäß Anspruch 1, dadurch gekennzeichnet, daß man bei der Durchführung der zweiten Stufe die Diazotierung bei Temperaturen zwischen 0°C und 20°C vornimmt und die thermische Hydrolyse bei Temperaturen zwischen 50°C und 100°C vornimmt.

## Claims

1. Process for the preparation of hydroxy-benzaldoxime O-ethers of the formula

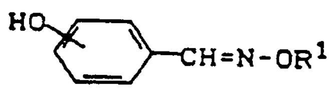

(I)

in which
$R^1$ represents alkyl,
characterized in that

a) in a first step, nitro-benzaldoxime O-ethers of the formula

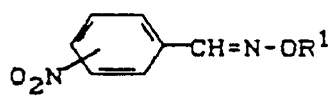

(II)

in which
$R^1$ has the abovementioned meaning,
are hydrogenated using the calculated amount of hydrogen in the presence of a catalyst and in the presence of a diluent, and

b) in a second step, the amino-benzaldoxime O-ethers of the formula

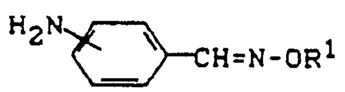

(III)

in which
$R^1$ has the abovementioned meaning,
thus obtained are reacted with a diazotizing agent in acidic, aqueous solution, and the resultant diazonium salts of the formula

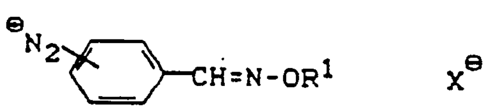

(IV)

in which
$X^{\ominus}$ represents an equivalent of an inorganic anion, and
$R^1$ has the abovementioned meaning,
are thermally hydrolysed in acidic, aqueous solution without intermediate isolation.

2. Process according to Claim 1, characterized in that the starting material employed is a nitro-benzaldoxime O-ether of the formula (II) in which $R^1$ represents straight-cain or branched alkyl having 1 to 20 carbon atoms.

3. Process according to Claim 1, characterized in that the starting material employed is a nitro-benzaldoxime O-ether of the formula (II) in which $R^1$ represents straight-chain or branched alkyl having 1 to 10 carbon atoms.

4. Process according to Claim 1, characterized in that the starting material employed is a nitro-benzaldoxime O-ether of the formula (II) in which $R^1$ represents straight-chain or branched alkyl having 1 to 4 carbon atoms.

5. Process according to Claim 1, characterized in that the starting material employed is 4-nitrobenzaldoxime O-methyl ether.

6. Process according to Claim 1, characterized in that the reaction accelerators employed are nickel-containing catalysts.

7. Process according to Claim 1, characterized in that the diazotizing agent employed when carrying out the second step is sodium nitrite, potassium nitrite, ammonium nitrite or nitrosylsulphuric acid.

8. Process according to Claim 1, characterized in that the acidifying agent employed when carrying out the second step is aqueous sulphuric acid.

9. Process according to Claim 1, characterized in that the first step is carried out at temperatures between 0°C and 100°C.

10. Process according to Claim 1, characterized in that, when carrying out the second step, the diazotization is carried out at temperatures between 0°C and 20°C and the thermal hydrolysis is carried out at temperatures between 50°C and 100°C.

## Revendications

1. Procédé de production de O-éthers d'hydroxybenzaldoxime de formule

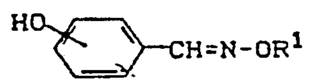

(I)

dans laquelle
$R^1$ est un groupe alkyle,
caractérisé en ce que

a) on hydrogène dans <u>une première étape</u> un O-éther de nitrobenzaldoxime de formule

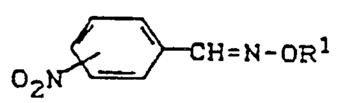

(II)

dans laquelle
$R^1$ a la définition indiquée ci-dessus, avec la quantité calculée d'hydrogène en présence d'un catalyseur de même qu'en présence d'un diluant et
b) on fait réagir dans <u>une seconde étape</u> le O-éther d'aminobenzaldoxime ainsi obtenu de formule

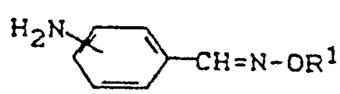

(III)

dans laquelle
$R^1$ a la définition indiquée ci-dessus,
avec un agent de diazotation en solution aqueuse acide, et on hydrolyse thermiquement les sels de diazonium produits de formule

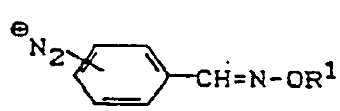

$X^{\ominus}$ (IV)

dans laquelle
$X^{\theta}$ représente un équivalent d'un anion inorganique et
$R^1$ a la définition indiquée ci-dessus, sans isolement intermédiaire, en solution aqueuse acide.

2. Procédé suivant la revendication 1, caractérisé en ce qu'on utilise comme composés de départ des O-éthers de nitrobenzaldoxime de formule (II) dans lesquels R représente un groupe alkyle à chaîne droite ou à

chaîne ramifiée ayant 1 à 20 atomes de carbone.

3. Procédé suivant la revendication 1, caractérisé en ce qu'on utilise comme composés de départ des O-éthers de nitrobenzaldoxime de formule (II) dans lesquels $R^1$ est un groupe alkyle à chaîne droite ou à chaîne ramifiée ayant 1 à 10 atomes de carbone.

4. Procédé suivant la revendication 1, caractérisé en ce qu'on utilise comme composés de départ des O-éthers de nitrobenzaldoxime de formule (II) dans lesquels $R^1$ est un groupe alkyle à chaîne droite ou à chaîne ramifiée ayant 1 à 4 atomes de carbone.

5. Procédé suivant la revendication 1, caractérisé en ce qu'on utilise comme composé de départ le O-méthyléther de 4-nitrobenzaldoxime.

6. Procédé suivant la revendication 1, caractérisé en ce qu'on utilise des catalyseurs contenant du nickel comme accélérateurs de réaction.

7. Procédé suivant la revendication 1, caractérisé en ce qu'on utilise le nitrite de sodium, le nitrite de potassium, le nitrite d'ammonium ou l'acide nitrosylsulfurique comme agent de diazotation pour la conduite de la seconde étape.

8. Procédé suivant la revendication 1, caractérisé en ce qu'on utilise l'acide sulfurique aqueux comme agent acidifiant pour la conduite de la seconde étape.

9. Procédé suivant la revendication 1, caractérisé en ce qu'on opère à des températures comprises entre 0°C et 100°C dans la mise en oeuvre de la première étape.

10. Procédé suivant la revendication 1, caractérisé en ce que, dans la mise en oeuvre de la seconde étape, on effectue la diazotation à des températures comprises entre 0°C et 20°C, et on conduit l'hydrolyse thermique à des températures comprises entre 50°C et 100°C.